# EUROPÄISCHE PATENTANMELDUNG

(11) **EP 2 233 922 A1**
(43) Veröffentlichungstag der Anmeldung: **29.09.2010**
(21) Anmeldenummer: 09156482.3
(22) Anmeldetag: 27.03.2009
(51) Int. Cl.: G01N 33/28

(54) **Verfahren zur Online-Überwachung der in Isolierflüssigkeiten von Hochspannungsanlagen gelösten atmosphärischen Gase und Vorrichtung zur Durchführung des Verfahrens**

(71) Anmelder: Industrie- und Umweltlaboratorium Vorpommern GmbH, 17489 Greifswald (DE)
(72) Erfinder: Bräsel, Eckhard, Dr., 17493, Greifswald (DE); Sasum, Ute, Dr., 17491, Greifswald (DE)
(74) Vertreter: Garrels, Sabine

(57) **Zusammenfassung**

Verfahren zur Online-Überwachung der in Isolierflüssigkeiten von Hochspannungsanlagen gelösten atmosphärischen Gase ohne den Einsatz eines Gasanalysators, mit dem Ziel einer breiteren Anwendung.
Darüber hinaus wird eine Vorrichtung zur Durchführung des Verfahrens offenbart.
Aufgabe ist es, die Bestimmung der in Isolierflüssigkeiten von Hochspannungsanlagen gelösten atmosphärischen Gase in das Online-Monitoring ausgewählter Fehlergase so einzubeziehen, dass nützliche Effekte sowohl für die Bestimmung als auch die Bewertung der gelösten Gase entstehen. Insbesondere soll dabei der Lösungsdruck auch darstellbar sein.
Bei seiner Bestimmung kann vorteilhaft die Erfahrung mit der Berechnung von Lösungsdrücken aus Vollanalysen berücksichtigt werden, die besagt, dass sich der Lösungsdruck näherungsweise durch die geringöllöslichen Luftbestandteile Stickstoff und Sauerstoff ergibt. Für diese beiden Hauptbestandteile des Lösungsdrucks kann erfindungsgemäß die Analyse beider Gase durch eine kombinierte Messung von nur Gleichgewichtsdrücken und -temperaturen unter Berücksichtigung der jeweiligen Ausgangsdaten ersetzt werden. Für genauere Bestimmungen des Lösungsdrucks ist eine Druckkorrektur durch die analysierten Fehlergase erforderlich.

## Beschreibung

Die Erfindung betrifft ein Verfahren zur Online-Überwachung der in Isolierflüssigkeiten von Hochspannungsanlagen gelösten atmosphärischen Gase ohne den Einsatz eines Gasanalysators, mit dem Ziel einer breiteren Anwendung. Die Erfindung betrifft darüber hinaus eine Vorrichtung zur Durchführung des Verfahrens.

### Stand der Technik

Nach DIN EN 60567 werden im Öl von elektrischen Betriebsmitteln neun Einzelgase im Labor bestimmt. Neben den sieben Fehlergasen sind das auch die beiden atmosphärischen Gase Stickstoff und Sauerstoff. Unterschiedliche Probenahme- und Extraktionstechniken werden beschrieben. Die Analyse der extrahierten Gase erfolgt einheitlich gaschromatographisch. Dabei wird das extrahierte Gas vollständig aufgetrennt und komponentenselektiv detektiert.

Vom Prinzip her können Labormessplätze automatisiert und klimatisiert auch online zum Einsatz kommen. Die Anschaffungs- und Wartungskosten sind aber sehr hoch. Alternativen werden mit IR- bzw. Photoakustik-Detektion für mehratomige Gase angeboten. Für Wasserstoff werden komplettierend Einzelsensoren eingesetzt, teilweise auch für Sauerstoff. Der Stickstoffgehalt wird aus dem Gesamtgasgehalt nach der Subtraktion aller bestimmten Gase berechnet. Die Gasanalysatoren können auch kombiniert sein mit speziellen Online-Techniken zur Gasextraktion, die ein Online-Gleichgewichtsgas kontinuierlich (DE 10 122 173) bzw. periodisch (DE 198 33 601 C1) bereitstellen, indem der Lösungsdruck direkt gemessen wird und die Gase analysiert werden können. Es sind auch Techniken bekannt, die für die Gasbereitstellung Membranen benutzen. Häufig werden die atmosphärischen Gase online unberücksichtigt gelassen. Insbesondere ist das der Fall beim Online-Monitoring ausgewählter Fehlergase, wo bei Online-Einzelgas- bzw. Schlüsselgas-Analysatoren wegen des Kostendrucks darauf verzichtet wird. Ursache ist aber auch die fehlende Aufklärung über die Bedeutung der atmosphärischen Gase in isolierflüssigkeitsbeaufschlagten Hochspannungsanlagen.

Nach DIN EN 60599 zur Bewertung von im Öl von elektrischen Betriebsmitteln gelösten Gasen werden die atmosphärischen Gase zur Bewertung der Dichtheit von geschlossenen Transformatoren und als Sauerstoff/Stickstoff-Quotient für die Bewertung der Öloxidation benutzt. Letzteres muss auch unbedingt auf die Festisolation erweitert werden. Da die atmosphärischen Gase über den Lösungsdruck auch die Wahrscheinlichkeit des Auftretens von Gasalarmen sowie über Rücksättigungsmessungen die Offenheit eines Transformators, was für die Bewertung aller Gase wichtig ist, zu bestimmen gestatten, wird ihre Bedeutung noch weiter hervorgehoben.

### Darstellung der Erfindung

Aufgabe der Erfindung ist es, die Bestimmung der in Isolierflüssigkeiten von Hochspannungsanlagen gelösten atmosphärischen Gase in das Online-Monitoring ausgewählter Fehlergase so einzubeziehen, dass nützliche Effekte sowohl für die Bestimmung als auch die Bewertung der gelösten Gase entstehen. Insbesondere soll dabei auch der Lösungsdruck darstellbar sein.

Bei seiner Bestimmung kann vorteilhaft die Erfahrung mit der Berechnung von Lösungsdrücken aus Vollanalysen berücksichtigt werden, die besagt, dass sich der Lösungsdruck näherungsweise durch die geringöllöslichen Luftbestandteile Stickstoff und Sauerstoff ergibt. Für diese beiden Hauptbestandteile des Lösungsdrucks kann erfindungsgemäß die Analyse beider Gase durch eine kombinierte Messung von nur Gleichgewichtsdrücken und -temperaturen ersetzt werden. Für genauere Bestimmungen des Lösungsdrucks ist eine Druckkorrektur durch die analysierten Fehlergase erforderlich.

Erfindungsgemäß wird die Aufgabe durch die Merkmale der Ansprüche 1. und 5 gelöst. Vorteilhafte Ausgestaltungen ergeben sich aus den Unteransprüchen.

Das Verfahren zur Online-Überwachung der in Isolierflüssigkeiten von Hochspannungsanlagen gelösten atmosphärischen Gase auf Basis der Einstellung des Lösungsgleichgewichtes zwischen den zwei Phasen Isolierflüssigkeit und Luft oder Isolierflüssigkeit und Inertgas und Erfassung der Ausgangs- und Gleichgewichtsdaten durch Messung von Druck und Temperatur ist **dadurch gekennzeichnet, dass** dieser erste Gleichgewichtszustand nach dem Erneuern der Gasphase mit neuer Luft oder neuem Inertgas definiert gestört und danach in einen zweiten Gleichgewichtszustand überführt wird, so dass erneut die Ausgangs- und Gleichgewichtsdaten durch Messung von Druck und Temperatur erfasst werden, um so mittels des Henry-Dalton'schen Gesetzes nur mit den jeweils gemessenen Drücken und Temperaturen über ein Gleichungssystem die Konzentrationen der atmosphärischen Gase in der Isolierflüssigkeit ausreichend genau zu bestimmen.

Die Vorrichtung ist **dadurch gekennzeichnet, dass** ein Austauschgefäß mit einem Probennahmepunkt des Transformators, einem Ölsammelgefäß, einem Gasvorratsgefäß, einem Niveaugefäß und einer Gaspumpe verbunden ist und Temperatursensoren jeweils im Austauschgefäß und im Gasvorratsgefäß sowie ein Drucksensor im Gasvorratsgefäß angeordnet sind, welche ihre Daten an eine Steuer- und Auswerteeinheit liefern.

Für die erfolgreiche Realisierung der Erfindung ist von Bedeutung, dass in ein und derselben Messeinrichtung zwischen Isolierflüssigkeit und Luft bzw. Inertgas nacheinander sich ein erstes Lösungsgleichgewicht und durch einmalig vollständiges oder teilweises Erneuern der Luft bzw. des Inertgases sich ein zweites Lösungsgleichgewicht nach dem Henry-Dalton'schen Gesetz einstellen sowie jeweils im Ausgangs- und Gleichgewichtszustand Druck und Temperatur gemessen werden. Durch Luft- bzw. Inertgaskorrektur wird so ein Gleichungssystem erhalten, das nur aus Druck- und Temperaturmessungen die Bestimmung der in Isolierflüssigkeiten von Hochspannungsanlagen gelösten atmosphärischen Gase gestattet. Für höhere Genauigkeiten werden in der ersten Gleichgewichtsgasphase ausgewählte Fehlergase analysiert und berücksichtigt sowie auch in die Berechnung des Lösungsdrucks einbezogen.

### Kurze Beschreibung der Abbildungen

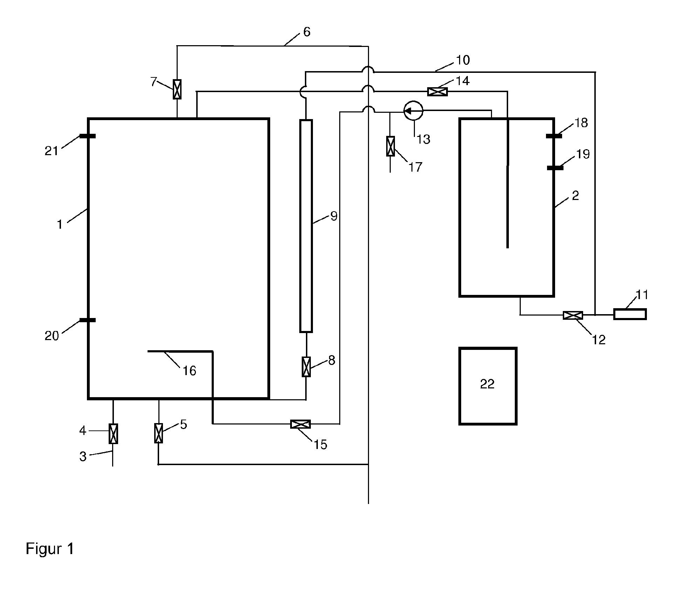

Die Erfindung soll nachfolgend anhand eines Ausführungsbeispiels an einem Öltransformator näher erläutet werden. Die dazugehörige Zeichnung zeigt in Fig. 1 die erfindungsgemäße Messeinrichtung.

### Ausführung der Erfindung

Die periodisch arbeitende Messeinrichtung zur Überwachung der im Öl gelösten atmosphärischen Gase, die am Fuße des Transformators installiert ist, besteht im wesentlichen aus einem Austauschgefäß 1, einem nicht weiter dargestellten Ölsammelgefäß, das gasraumseitig vorzugsweise eine Verbindung zum Gasraum des Ausdehnungsgefäßes des Transformators besitzt, und einem Gasvorratsgefäß 2. Vom Boden des Austauschgefäßes 1 führt eine Füllleitung 3 mit Schaltventil 4 zu einem Probenahmepunkt des Transformators. Das Ölsammelgefäß besitzt eigene Hilfsmittel, um das Öl in den Transformator zurückzuführen.

Vom Boden des Austauschgefäßes 1 führt eine Entleerungsleitung mit Schaltventil 5 direkt in eine Spülleitung 6, die vom Kopf des Austauschgefäßes 1 mit Schaltventil 7 vertikal bis zum Boden des Ölsammelgefäßes führt. Seitlich unten an dem Austauschgefäß 1 setzt ein Schaltventil 8 an, das zum parallel angeordneten Niveaugefäß 9 führt, von welchem am oberen Ende eine Leitung 10 zu einem Luftreservoir 11, was ständig unter Atmosphärendruck zur Verfügung steht, führt.

Vom Kopf des Austauschgefäßes 1 führt ein Schaltventil 14 in das Gasvorratsgefäß 2, von dem über eine Gaspumpe 13 und ein Schaltventil 15 eine Verbindung zum Gaseintrittsstutzen 16 am Boden des Austauschgefäßes 1 führt. Über ein Schaltventil 17 besteht von der Gaspumpe 13 auch eine Verbindung zur Atmosphäre. Das Gasvorratsgefäß 2 besitzt jeweils einen Temperatur- 19 bzw. Drucksensor 18. Weitere Temperatursensoren 20 und 21 sind in dem Austauschgefäß 1 angebracht.

Die Messeinrichtung wird über eine Software in der Steuer- und Auswerteeinheit 22 gestartet. Alle Hähne und Schaltventile sind geschlossen. In allen Gefäßen befindet sich trockene atmosphärische Luft.

Jetzt wird das Schaltventil 4 an der Füllleitung und das Schaltventil 7 an der Spülleitung geöffnet, womit der Spül- und Füllvorgang für das Austauschgefäß 1 eingeleitet wird. Dieser ist dann beendet, wenn im Ölsammelgefäß ein oberer Höhenstandssensor erreicht wird und die beiden Schaltventile 4 und 7 wieder geschlossen sind. Bei Bedarf kann ein nächster Spülschritt des Austauschgefäßes 1 mit Entleerung des Ölsammelgefäßes analog wiederholt werden. Nun öffnen für eine vorgegebene Zeit die Schaltventile 8 am Niveaugefäß 9, sowie 12 und 14, in deren Folge sich zwischen dem Austauschgefäß 1 und dem Niveaugefäß 9 ein gleicher Ölspiegelstand einstellt. Nach Ablauf der vorgegebenen Zeit schließen die Schaltventile 8 und 12 wieder, Schaltventil 14 bleibt offen. Nun werden die Ausgangsdaten der Temperatursensoren 19, 20 und 21 sowie vom Drucksensor 18 erfasst. Danach öffnet Schaltventil 15, welches mit dem Gaseintrittsstutzen 16 verbunden ist, und die Gaspumpe 13 startet für eine vorgegebene Zeit, die ausreicht, um das Lösungsgleichgewicht zwischen dem Originalöl in dem Austauschgefäß 1 und dem erfassten Luftvolumen herzustellen. Nach Ablauf der Zeit werden die Gleichgewichtsdaten analog den Ausgangsdaten erfasst und die Schaltventile 14 und 15 geschlossen. In diesem Zustand ist es möglich, in bekannter Weise ausgewählte Fehlergase im Gasvorratsgefäß 2 zu bestimmen. Zum Erneuern der Luft öffnen das Schaltventil 17 an der Gaspumpe sowie das Schaltventil 12. Mit startender Gaspumpe 13 wird trockene Luft aus dem Luftreservoir 11 durch das Gasvorratsgefäß 2 gespült. Nach einer vorgegebenen Zeit ist die Luftspülung abgeschlossen, indem die Gaspumpe 13 stoppt und die Schaltventile 12 und 17 schließen. Nun werden zum 2. Mal die Ausgangsdaten der Temperatursensoren 19, 20, 21 sowie des Drucksensors 18 über die Steuer- und Auswerteeinheit 22 erfasst. Dann öffnen die Schaltventile 15, welches mit dem Gaseintrittsstutzen 16 verbunden ist, und 14, womit ein Druckausgleich mit dem Gasraum des Austauschgefäßes 1 stattfindet. Nun startet für eine vorgegebene Zeit die Gaspumpe 13. Nach Ablauf der Zeit werden die Gleichgewichtsdaten analog den Ausgangsdaten erfasst.

In der Steuer- und Auswerteeinheit 22 liegen nun alle Daten für die Berechnung 2 vor. Durch die Messung ausgewählter Fehlergasgehalte im ersten Gleichgewichtszustand sind nur noch die beiden atmosphärischen Gase zu bestimmen. Das erfolgt über die beiden Gleichgewichtsdruckmessungen, indem ein Gleichungssystem mit den beiden unbekannten Konzentrationen für Sauerstoff und Stickstoff im Öl des Transformators zu lösen ist.

Abschließend erfolgt die Berechnung des Lösungsdrucks. Entsprechend der Aufgabenstellung lassen sich nun folgende Bewertungen vornehmen:
- Sättigungszustand des Öles anhand des Lösungsdrucks;
- Rücksättigungsverläufe anhand von Stickstoff;
- Dichtheit von Hermetikabschlüssen anhand von Stickstoff;
- Auffälligkeit und deren Aktualität anhand der ausgewählten Fehlergasgehalte.

Ein Messzyklus ist abgeschlossen, wenn die Messvorrichtung ölentleert und luftgespült ist. Zuerst öffnen dazu die Schaltventile 12 und 14 sowie 5 und 8, womit das Austauschgefäß 1 einschließlich Niveaugefäß 9 leer läuft. Danach werden die Schaltventile 5, 8, und 14 geschlossen. Schaltventil 17 öffnet und die Gaspumpe 13 startet für eine vorgegebene Zeit. Mit dem Abschalten der Gaspumpe 13 werden auch die Schaltventile 12 und 17 wieder geschlossen. Parallel zum Luftspülen erfolgt das Absenken des Ölstandes im Ölsammelgefäß. Damit ist ein Messzyklus komplett abgeschlossen. Über die Steuer- und Auswerteeinheit 22 erfolgen die Wiederholungen im vorgegebenen Zeitabstand aus diesem Grundzustand heraus.

Der gesamte Gastrakt der Messvorrichtung kann statt mit Luft auch analog mit einem Inertgas betrieben werden. Das Inertgasreservoir ist dann 11.

Zusätzlich kann vom Gasdom des Buchholzrelais eine Verbindung zum Austauschgefäß 1 geschaffen werden, die in bekannter Weise im Falle eines Gasalarms den Gastransport in das Austauschgefäß 1 gestattet, um so eine Schnellanalyse ausgewählter Fehlergase zu ermöglichen.

Außerdem kann aus der Füllleitung 3 repräsentatives Öl in Probenahmegefäße gespült werden, wobei die Entlüftung und der Überlauf in das nicht näher benannte Ölsammelgefäß erfolgt. Weiterhin können Sensoren für ausgewählte Fehlergase mit dem Gasvorratsgefäß 2 verbunden sein.

### Bezugszeichen

- 1: Austauschgefäß
- 2: Gasvorratsgefäß
- 3: Füllleitung
- 4: Schaltventil Füllleitung
- 5: Schaltventil Entleerungsleitung
- 6: Spülleitung
- 7: Schaltventil Spülleitung
- 8: Schaltventil Niveaugefäß
- 9: Niveaugefäß
- 10: Leitung
- 11: Luft- oder Inertgasreservoir
- 12: Schaltventil
- 13: Gaspumpe
- 14: Schaltventil
- 15: Schaltventil Gaseintrittsstutzen
- 16: Gaseintrittsstutzen
- 17: Schaltventil Gaspumpe
- 18: Drucksensor des Gasvorratsgefäßes 2
- 19: Temperatursensor des Gasvorratsgefäßes 2
- 20: Temperatursensor des Austauschgefäßes 1
- 21: Temperatursensor des Austauschgefäßes 1
- 22: Steuer- und Auswerteeinheit

## Patentansprüche

1. Verfahren zur Online-Überwachung der in Isolierflüssigkeiten von Hochspannungsanlagen gelösten atmosphärischen Gase auf Basis der Einstellung des Lösungsgleichgewichtes zwischen den zwei Phasen Isolierflüssigkeit und Luft oder Isolierflüssigkeit und Inertgas und Erfassung der Ausgangs- und Gleichgewichtsdaten durch Messung von Druck und Temperatur **dadurch gekennzeichnet, dass**
dieser erste Gleichgewichtszustand nach dem Erneuern der Gasphase mit neuer Luft oder neuem Inertgas definiert gestört und danach in einen zweiten Gleichgewichtszustand überführt wird, so dass erneut die Ausgangs- und Gleichgewichtsdaten durch Messung von Druck und Temperatur erfasst werden, um so mittels des Henry-Dalton'schen Gesetzes nur mit den jeweils gemessenen Drücken und Temperaturen über ein Gleichungssystem die Konzentrationen der atmosphärischen Gase in der Isolierflüssigkeit ausreichend genau zu bestimmen.

2. Verfahren nach Anspruch 1 **dadurch gekennzeichnet, dass**
über eine Steuer- und Auswerteeinheit (31) in einem vorgegebenen Zeitabstand der Messzyklus wiederholt wird.

3. Verfahren nach Anspruch 1 oder 2 **dadurch gekennzeichnet, dass**
für besonders genaue Bestimmungen der Konzentrationen der atmosphärischen Gase in der Isolierflüssigkeit die gemessenen Gleichgewichtsdrücke um einen nach dem Henry-Dalton'schen Gesetz berechenbaren Anteil von ausgewählten online überwachten Fehlergasen korrigiert werden.

4. Verfahren nach einem der vorhergehenden Ansprüche 1 bis 3 **dadurch**
**gekennzeichnet, dass**
aus den jeweils gemessenen Gleichgewichtsdrücken und Gleichgewichtstemperaturen bestimmten Konzentrationen der atmosphärischen Gase in der Isolierflüssigkeit der Lösungsdruck ausreichend genau berechnet wird.

5. Vorrichtung zur Online-Überwachung der in Isolierflüssigkeiten von Hochspannungsanlagen gelösten atmosphärischen Gase **dadurch**
**gekennzeichnet,**
**dass** ein Austauschgefäß (1) mit einem Probennahmepunkt des Transformators, einem Ölsammelgefäß, einem Gasvorratsgefäß (2), einem Niveaugefäß (9) und einer Gaspumpe (13) verbunden ist und Temperatursensoren (20) und (21) des Austauschgefäßes (1) und (19) vom Gasvorratsgefäß (2) sowie ein Drucksensor (18) im Gasvorratsgefäß (2) angeordnet sind, welche ihre Daten an eine Steuer- und Auswerteeinheit (22) liefern.

6. Vorrichtung nach Anspruch 5 **dadurch gekennzeichnet, dass**
eine Entleerungsleitung mit Schaltventil (5) von dem Austauschgefäß (1) direkt in eine Spülleitung (6) mündet, welche vom oberen Ende des Austauschgefäßes (1) über ein Schaltventil (7) bis zum Boden des Ölsammelgefäßes führt.

7. Vorrichtung nach einem der Ansprüche 5 bis 6 **dadurch gekennzeichnet, dass** das Niveaugefäß (9) über ein Schaltventil (8) mit dem Austauschgefäß (1) verbunden ist und über eine Leitung (10) zu einem Luftreservoir führt.

8. Vorrichtung nach einem der Ansprüche 5 bis 7 **dadurch gekennzeichnet, dass** das Austauschgefäß (1) über ein Schaltventil (14) mit dem Gasvorratsgefäß (2) verbunden ist, von welchem über eine Gaspumpe (13) und ein Schaltventil (15) eine Verbindung zum Gaseintrittsstutzen (16) am Boden des Austauschgefäßes (1) führt.

9. Vorrichtung nach einem der Ansprüche 5 bis 8 **dadurch gekennzeichnet, dass** die Gaspumpe (13) über ein Schaltventil (17) eine Verbindung zur Atmosphäre hat.

## Geänderte Patentansprüche

### Geänderte Patentansprüche gemäss Regel 137(2) EPÜ.

**1.** Verfahren zur Online-Überwachung der in Isolierflüssigkeiten von Hochspannungsanlagen gelösten atmosphärischen Gase, insbesondere Stickstoff und Sauerstoff, auf Basis der Einstellung des Lösungsgleichgewichtes zwischen den zwei Phasen Isolierflüssigkeit und Luft oder Isolierflüssigkeit und Inertgas und Erfassung der Ausgangs- und Gleichgewichtsdaten durch Messung von Druck und Temperatur **dadurch gekennzeichnet, dass**
dieser erste Gleichgewichtszustand nach dem Erneuern der Gasphase mit neuer Luft oder neuem Inertgas definiert gestört und danach in einen zweiten Gleichgewichtszustand überführt wird, so dass erneut die Ausgangs- und Gleichgewichtsdaten durch Messung von Druck und Temperatur erfasst werden, um so mittels des Henry-Dalton'schen Gesetzes nur mit den jeweils gemessenen Drücken und Temperaturen über ein Gleichungssystem die Konzentrationen der atmosphärischen Gase in der Isolierflüssigkeit ausreichend genau zu bestimmen.

**2.** Verfahren nach Anspruch 1 **dadurch gekennzeichnet, dass** über eine Steuer- und Auswerteeinheit (31) in einem vorgegebenen Zeitabstand der Messzyklus wiederholt wird.

**3.** Verfahren nach Anspruch 1oder 2 **dadurch gekennzeichnet, dass** für besonders genaue Bestimmungen der Konzentrationen der atmosphärischen Gase in der Isolierflüssigkeit die gemessenen Gleichgewichtsdrücke um einen nach dem Henry-Dalton'schen Gesetz berechenbaren Anteil von ausgewählten online überwachten Fehlergasen korrigiert werden.

**4.** Verfahren nach einem der vorhergehenden Ansprüche 1 bis 3 **dadurch gekennzeichnet, dass**
aus den jeweils gemessenen Gleichgewichtsdrücken und Gleichgewichtstemperaturen bestimmten Konzentrationen der atmosphärischen Gase in der Isolierflüssigkeit der Lösungsdruck ausreichend genau berechnet wird.

**5.** Vorrichtung zur Online-Überwachung der in Isolierflüssigkeiten von Hochspannungsanlagen gelösten atmosphärischen Gase, insbesondere Stickstoff und Sauerstoff, auf Basis der Einstellung des Lösungsgleichgewichtes zwischen den zwei Phasen Isolierflüssigkeit und Luft oder Isolierflüssigkeit und Inertgas und Erfassung der Ausgangs- und Gleichgewichtsdaten durch Messung von Druck und Temperatur **dadurch gekennzeichnet,**
**dass** ein Austauschgefäß (1) mit einem Probennahmepunkt des Transformators, einem Ölsammelgefäß, einem Gasvorratsgefäß (2), einem Niveaugefäß (9) und einer Gaspumpe (13) verbunden ist und Temperatursensoren (20) und (21) des Austauschgefäßes (1) und (19) vom Gasvorratsgefäß (2) sowie ein Drucksensor (18) im Gasvorratsgefäß (2) angeordnet sind, welche ihre Daten an eine Steuer- und Auswerteeinheit (22) liefern.

**6.** Vorrichtung nach Anspruch 5 **dadurch gekennzeichnet, dass** eine Entleerungsleitung mit Schaltventil (5) von dem Austauschgefäß (1) direkt in eine Spülleitung (6) mündet, welche vom oberen Ende des Austauschgefäßes (1) über ein Schaltventil (7) bis zum Boden des Ölsammelgefäßes führt.

**7.** Vorrichtung nach einem der Ansprüche 5 bis 6 **dadurch gekennzeichnet, dass** das Niveaugefäß (9) über ein Schaltventil (8) mit dem Austauschgefäß (1) verbunden ist und über eine Leitung (10) zu einem Luftreservoir führt.

**8.** Vorrichtung nach einem der Ansprüche 5 bis 7 **dadurch gekennzeichnet, dass** das Austauschgefäß (1) über ein Schaltventil (14) mit dem Gasvorratsgefäß (2) verbunden ist, von welchem über eine Gaspumpe (13) und ein Schaltventil (15) eine Verbindung zum Gaseintrittsstutzen (16) am Boden des Austauschgefäßes (1) führt.

**9.** Vorrichtung nach einem der Ansprüche 5 bis 8 **dadurch gekennzeichnet, dass** die Gaspumpe (13) über ein Schaltventil (17) eine Verbindung zur Atmosphäre hat.
